# EUROPEAN PATENT APPLICATION

(11) **EP 4 339 879 A1**
(43) Date of publication of application: **20.03.2024**
(21) Application number: 22196388.7
(22) Date of filing: 19.09.2022
(51) Int. Cl.: G06T 5/00, G06T 5/50, G06T 7/70

(54) **ANATOMY MASKING FOR MRI**

(71) Applicant: Siemens Healthineers AG, 91301 Forchheim (DE); Sirona Dental Systems GmbH, 64625 Bensheim (DE); Dentsply Sirona Inc., York, PA 17401 (US)
(72) Inventor: GREISER, Andreas, Dr., 91054 Erlangen (DE); ULRICI, Johannes, Dr., 64285 Darmstadt (DE); BURZAN, Kim, 69488 Birkenau (DE); LAUER, Lars, Dr., 91077 Neunkirchen (DE); RINCK, Daniel, 91301 Forchheim (DE); HAYES, Carmel, Dr., 80634 München (DE); KRÜGER, Gunnar, Dr., 4102 Binningen (CH)
(74) Representative: Siemens Healthineers Patent Attorneys

(57) **Abstract**

Techniques for masking one or more regions surrounding an anatomical region of interest for each of one or more MRI images obtained from MRI imaging data are disclosed. The one or more regions surrounding the anatomical region of interest are masked based on control data determined for identifying the anatomical region of interest in the MRI imaging data. The MRI imaging data are obtained during an MRI exam of a patient for a measurement volume including the anatomical region of interest. The anatomical region of interest is ascertained before performing the MRI exam.

## Description

Various examples of the invention generally relate to magnetic resonance (MR) imaging (MRI). Various examples specifically relate to masking one or more regions surrounding an anatomical region of interest for MRI images.

MRI plays an important role in clinical medicine, and it can visualize human organs and tissues to help follow-up diagnosis. During an MRI exam of a patient, acquired spatial MRI images depict not only an anatomical region of interest but also one or more regions surrounding the anatomical region of interest. The one or more regions surrounding the anatomical region of interest may be not of interest to a specific clinical/medical practitioner, e.g., a dentist, a cardiologist, a surgeon, a gastroenterologist, a nephrologist, a neurologist, or an oncologist.

For example, when an MRI exam is performed to image the teeth of a patient, parts of the brain of the patient may be shown in the reconstructed/acquired MRI images of the MRI exam. Such parts of the brain are not of interest to a dentist and may increase the workload of the dentist to diagnose the MRI images. Further, when a larger head volume or parts of the brain are included in the scan, the risk of incidental or missed findings is increased since the clinicians e.g. dentists interpreting the scans are not trained to diagnose anatomy outside their area of expertise.

Patent application US 2021/0158563 A1 discloses methods to adjust the spatial coverage of MR measurement with regard to the dental region to be imaged, e.g., comprising an iterative adjustment of a field of view of the MR measurement based on a body model of the patient and the sensor signal indicative of the position and/or posture of the patient. Furthermore, the patent application discloses to employ of a dedicated scanner configured to acquire MR image data of a dental region of a patient, e.g. that the magnetic resonance imaging device is configured to perform an MR measurement of a teeth region and/or a jaw region of the patient. Thus, an imaging volume of the MRI device may be tailored to match a diagnostically relevant organ structure, such as a tooth, a jaw, a dental arch, or both dental arches of the patient.

However, existing techniques cannot automatically, rapidly, and accurately isolate or differentiate an anatomical region of interest for a specific clinical/medical practitioner from surrounding anatomical regions.

Therefore, a need exists for advanced techniques for MRI imaging. Specifically, a need exists for processing MRI imaging data to highlight an anatomical region of interest. A need exists for MRI imaging facilitating a specific diagnosis.

This need is met by the features of the independent claims. The features of the dependent claims define embodiments.

A computer-implemented method for processing MRI imaging data is provided. The method comprises ascertaining an anatomical region of interest associated with an MRI exam of a patient. The method further comprises obtaining MRI imaging data for a measurement volume including the anatomical region of interest and determining control data for identifying the anatomical region of interest in the MRI imaging data. The method also comprises, based on the control data, masking one or more regions surrounding the anatomical region of interest for each of one or more MRI images obtained from the MRI imaging data.

A computing device comprising a processor and a memory is provided. Upon loading and executing program code from the memory, the processor is configured to perform a method for processing MRI imaging data. The method comprises ascertaining an anatomical region of interest associated with an MRI exam of a patient. The method further comprises obtaining MRI imaging data for a measurement volume including the anatomical region of interest and determining control data for identifying the anatomical region of interest in the MRI imaging data. The method also comprises, based on the control data, masking one or more regions surrounding the anatomical region of interest for each of one or more MRI images obtained from the MRI imaging data.

An MRI scanner comprising a computing device is provided. The computing device comprises a processor and a memory. Upon loading and executing program code from the memory, the processor is configured to perform a method for processing MRI imaging data. The method comprises ascertaining an anatomical region of interest associated with an MRI exam of a patient. The method further comprises obtaining MRI imaging data for a measurement volume including the anatomical region of interest and determining control data for identifying the anatomical region of interest in the MRI imaging data. The method also comprises, based on the control data, masking one or more regions surrounding the anatomical region of interest for each of one or more MRI images obtained from the MRI imaging data.

A computer program product or a computer program or a computer-readable storage medium including program code is provided. The program code can be executed by at least one processor. Executing the program code causes the at least one processor to perform a method for processing MRI imaging data. The method comprises ascertaining an anatomical region of interest associated with an MRI exam of a patient. The method further comprises obtaining MRI imaging data for a measurement volume including the anatomical region of interest and determining control data for identifying the anatomical region of interest in the MRI imaging data. The method also comprises, based on the control data, masking one or more regions surrounding the anatomical region of interest for each of one or more MRI images obtained from the MRI imaging data.

It is to be understood that the features mentioned above and those yet to be explained below may be used not only in the respective combinations indicated, but also in other combinations or in isolation without departing from the scope of the invention.
FIG. 1 schematically illustrates an MRI scanner according to various examples.
FIG. 2 schematically illustrates a flowchart of a method according to various examples.
FIG. 3 schematically illustrates an exemplary masking MRI image according to various examples.
FIG. 4 schematically illustrates an exemplary MRI image according to various examples.
FIG. 5 schematically illustrates an exemplary masked MRI image according to various examples.
FIG. 6 schematically illustrates an exemplary workflow according to various examples.
FIG. 7 schematically illustrates a further exemplary workflow according to various examples.
FIG. 8 is a block diagram of a device according to various examples.

Some examples of the present disclosure generally provide for a plurality of circuits or other electrical devices. All references to the circuits and other electrical devices and the functionality provided by each are not intended to be limited to encompassing only what is illustrated and described herein. While particular labels may be assigned to the various circuits or other electrical devices disclosed, such labels are not intended to limit the scope of operation for the circuits and the other electrical devices. Such circuits and other electrical devices may be combined with each other and/or separated in any manner based on the particular type of electrical implementation that is desired. It is recognized that any circuit or other electrical device disclosed herein may include any number of microcontrollers, a graphics processor unit (GPU), integrated circuits, memory devices (e.g., FLASH, random access memory (RAM), read only memory (ROM), electrically programmable read only memory (EPROM), electrically erasable programmable read only memory (EEPROM), or other suitable variants thereof), and software which co-act with one another to perform operation(s) disclosed herein. In addition, any one or more of the electrical devices may be configured to execute a program code that is embodied in a non-transitory computer readable medium programmed to perform any number of the functions as disclosed.

In the following, embodiments of the invention will be described in detail with reference to the accompanying drawings. It is to be understood that the following description of embodiments is not to be taken in a limiting sense. The scope of the invention is not intended to be limited by the embodiments described hereinafter or by the drawings, which are taken to be illustrative only. The drawings are to be regarded as being schematic representations and elements illustrated in the drawings are not necessarily shown to scale. Rather, the various elements are represented such that their function and general purpose become apparent to a person skilled in the art. Any connection or coupling between functional blocks, devices, components, or other physical or functional units shown in the drawings or described herein may also be implemented by an indirect connection or coupling. A coupling between components may also be established over a wireless connection. Functional blocks may be implemented in hardware, firmware, software, or a combination thereof.

Hereinafter, techniques of MRI are described. MRI may be employed to obtain raw MR signals of magnetization of nuclear spins of a sample region of a patient (MR data), e.g., a region of the heart, the brain, the liver, the teeth, or the lung of a patient. The sample region defines a field of view (FOV). The FOV typically includes an anatomical region of interest and surrounding anatomical regions or background. The MR data are typically defined in k-space (k-space MRI imaging data). Based on the k-space MRI imaging data, MR images in the spatial domain can be determined (spatial domain MRI imaging data). As a general rule, the terms k-space MRI imaging data and spatial domain MRI imaging data may respectively denote a 2-D or 3-D k-space dataset and a 2-D or 3-D spatial dataset. Hereinafter, 2-D data will be used as exemplary data to describe various techniques of this disclosure, and the MRI imaging data may comprise k-space MRI imaging data and/or spatial domain MRI imaging data.

According to this disclosure, techniques for masking one or more regions surrounding an anatomical region of interest for each of one or more MRI images obtained from MRI imaging data are disclosed. The one or more regions surrounding the anatomical region of interest are masked based on control data determined for identifying the anatomical region of interest in the MRI imaging data. The MRI imaging data are obtained during an MRI exam of a patient for a measurement volume including the anatomical region of interest. The anatomical region of interest is ascertained before performing the MRI exam.

Such techniques are based on the finding that when performing an MRI exam of an anatomical region of interest, e.g., a dental region, a heart, a brain, or a kidney, it is advantageous that the one or more MRI images acquired using MR scanners show exclusively the anatomical region of interest. I.e., by masking the one or more regions surrounding the anatomical region of interest for each of the one or more MRI images obtained from MRI imaging data, the one or more images can exclude other anatomical regions (or surrounding anatomical regions of the anatomical region of interest) which are not of interest to a specific clinical/medical practitioner, e.g., a dentist, a cardiologist, or an oncologist.

The techniques disclosed herein adapt/process the images acquired using an MR scanner, e.g., a dental MR scanner so that the images may exclusively show the region of the patient, which is of interest to a dentist or a dental practitioner. I.e., the techniques disclosed herein can automatically, rapidly, and accurately isolate or segregate an anatomical region of interest for a specific clinical/medical practitioner from surrounding anatomical regions. Thus, the workload for the specific clinical/medical practitioner to diagnose the acquired images can be reduced and risks for the specific clinical/medical practitioner to miss pathologies in regions which she/he is not trained to diagnose can be reduced. For example, a dentist may not be trained for diagnosing pathologies in the brain.

As a general rule, the anatomical region of interest may be pre-defined or ascertained by a specific clinical/medical practitioner. Alternatively or optionally, the anatomical region of interest may be ascertained based on at least one of a purpose of the MRI exam, a type of a radiofrequency (RF) coil used during the MRI exam, and a capability of a physician who will give a diagnosis of the MRI exam.

According to various examples of this disclosure, the control data for identifying the anatomical region of interest may comprise at least one of a contour of the anatomical region of interest, one or more landmarks associated with the anatomical region of interest, a threshold associated with a distance to a respective one of one or more target objects comprised in the anatomical region of interest, and one or more masking MRI images (or mask). For example, the masking MRI images may be binary masks which could be placed over one or more MRI images obtained from the MRI imaging data to mask one or more regions surrounding the anatomical region of interest. Each of the binary masks may comprise a region coinciding with the anatomical region of interest in which all pixel values are set to 1 (or another predetermined value), and one or more regions respectively coinciding with the one or more regions surrounding the anatomical region of interest in which all pixel values are set to 0 (or another predetermined value). A masked MRI image exclusively showing the anatomical region of interest may be obtained by performing element-wise (or entry-wise) multiplication between a masking MRI image and a corresponding MRI image.

According to various examples of this disclosure, after masking one or more regions surrounding the anatomical region of interest for each of one or more MRI images obtained from the MRI imaging data, a graphical representation associated with the anatomical region of interest may be obtained based on the one or more masked MRI images and imaging parameters associated with an MRI scanner performing the MRI exam may be adjusted based on the graphical representation. Thus, the MRI scanner can be precisely controlled to acquire the followingup MRI imaging data of the MRI exam.

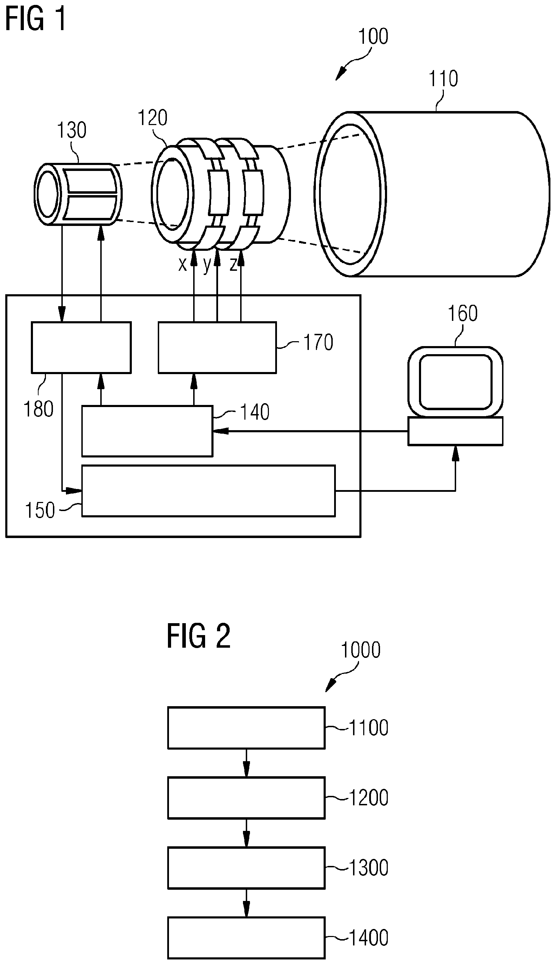

FIG. 1 illustrates aspects with respect to an MRI scanner 100. The MRI scanner 100 can be used to perform acquisitions of MRI imaging data for a measurement volume including an anatomical region of interest. Further, the MRI scanner 100 can be configured to reconstruct spatial domain MRI imaging data from k-space MRI imaging data and process the spatial domain MRI imaging data using techniques disclosed herein, e.g., masking one or more regions surrounding the anatomical region of interest for each of one or more MRI images obtained from the MRI imaging data. There are several principal components constituting an MRI scanner 100: the main magnet 110; a set of gradient coils 120 to provide switchable spatial gradients in the main magnetic field; RF coils 130 (or resonators) for the transmission and reception of radio frequency pulses; pulse sequence electronics 140 for programming the timing of transmission signals (excitation pulse, gradient signals); image reconstruction electronics 150 and a human machine interface 160 for viewing, manipulating, and storing images.

A common type of the main magnet 110 used in MRI systems is the cylindrical superconducting magnet (typically with a 1 meter bore size). The main magnet 110 can provide a main magnet field with a field strength varying from 0.5 T (21 MHz) to 3.0 T (128 MHz), even 9 T (383 MHz), along its longitudinal axis. The main magnetic field can align the magnetization of the nuclear spins of a patient along the longitudinal axis. The patient can be moved into the bore by means of a sliding table (not shown in FIG. 1).

The gradient coils 120 fit inside the bore of the main magnet 110 (after any active shimming coils, if present). The function of the gradient coils 120 is to provide a temporary change in the magnitude of the main magnetic field as a function of position in the bore of the main magnet 110. The gradient coils 120 provide a spatial encoding of the magnetic field strength, to thereby choose slices of the patient body for selective imaging. In this way, MRI can be tomographic - i.e., it can image slices. The gradient coils 120 also provide the means to spatially encode the voxels within a given image slice so that the individual echoes coming from each voxel can be discriminated and turned into an MR image. There are usually three orthogonal gradient coils, one for each of the physical x, y, and z directions. The gradients can be used for slice selection (slice-selection gradients), frequency encoding (readout gradients), and phase encoding along one or more phase-encoding directions (phase-encoding gradients). Hereinafter, the slice-selection direction will be defined as being aligned along the Z-axis; the readout direction will be defined as being aligned with the X-axis; and a first phase-encoding direction as being aligned with the Y-axis. A second phase-encoding direction may be aligned with the Z-axis. The directions along which the various gradients are applied are not necessarily in parallel with the axes defined by the gradient coils 120. Rather, it is possible that these directions are defined by a certain k-space trajectory which, in turn, can be defined by certain requirements of the respective MRI pulse sequence and/or based on anatomic properties of a patient. The gradient coils 120 usually coupled with the pulse sequence electronics 140 via gradient amplifiers 170.

RF pulses that are oscillating at the Larmor frequency applied around a sample causes nuclear spins to precess, tipping them toward the transverse plane. Once a spin system is excited, coherently rotating spins can induce RF currents (at the Larmor frequency) in nearby antennas, yielding measurable signals associated with the free induction decay and echoes. Thus, the RF coils 130 serve to both induce spin precession and to detect signals indicative of the precession of the nuclear spins. The RF coils 130 usually coupled with both the pulse sequence electronics 140 and the image reconstruction electronics 150 via RF electronics 180, respectively.

For creating such RF pulses, an RF transmitter (e.g., a part of the RF electronics 180) is connected via an RF switch (e.g., a part of the RF electronics 180) with the RF coils 130. Via an RF receiver (e.g., a part of the RF electronics 180), it is possible to detect the induced currents or signals by the spin system. In particular, it is possible to detect echoes; echoes may be formed by applying one or more RF pulses (spin echo) and/or by applying one or more gradients (gradient echo). The respectively induced currents or signals can correspond to raw MRI data in k-space; according to various examples, the MRI data can be processed using reconstruction techniques in order to obtain MRI images.

The human machine interface 160 might include at least one of a screen, a keyboard, a mouse, etc. By means of the human machine interface 160, a user input can be detected and output to the user can be implemented. For example, by means of the human machine interface 160, it is possible to select and configure the scanning pulse sequence, e.g., a gradientecho sequence or fast spin-echo sequence, graphically select the orientation of the scan planes to image, review images obtained, and change variables in the pulse sequence to modify the contrast between tissues. The human machine interface 160 is respectively connected to the pulse sequence electronics 140 and the image reconstruction electronics 150, such as an array processor, which performs the image reconstruction.

The pulse sequence electronics 140 may include a GPU and/or a CPU and/or an application-specific integrated circuit and/or a field-programmable array. The pulse sequence electronics 140 may implement various control functionality with respect to the operation of the MRI scanner 100, e.g. based on program code loaded from a memory. For example, the pulse sequence electronics 140 could implement a sequence control for time-synchronized operation of the gradient coils 120, both the RF transmitter and the RF receiver of the RF electronics 180.

The image reconstruction electronics 150 may include a GPU and/or a CPU and/or an application-specific integrated circuit and/or a field-programmable array. The image reconstruction electronics 150 can be configured to implement pre-processing and/or post-processing for reconstruction of MRI images. For example, the image reconstruction electronics 150 can be configured to perform either full sampling or undersampling of k-space MRI imaging data, and/or motion correction of the reconstructed spatial domain MRI imaging data.

The pulse sequence electronics 140 and the image reconstruction electronics 150 may be a single circuit, or two separate circuits.

The MRI scanner 100 may be connectable to a database (not shown in FIG. 1), such as a picture archiving and communication system (PACS) located within a local network of a hospital, for storing acquired MRI data, and/or, MRI images in k-space, and/or reconstructed MRI images in spatial domain, and/or post-processed MRI images, e.g., masked MRI images obtained using techniques disclosed herein.

Details with respect to techniques, such as the functioning of the MRI scanner 100, particularly the functioning of the image reconstruction electronics 150 or an additional computing device are described in connection with FIG. 2.

FIG. 2 is a flowchart of a method 1000 according to various examples. For example, the method 1000 according to FIG. 2 may be executed by the image reconstruction electronics 150 of the MRI scanner 100 according to the example of FIG. 1, e.g., upon loading program code from a memory. The method 1000 may be executed by the image reconstruction electronics 150 together with the pulse sequence electronics 140, for example, the image reconstruction electronics 150 may be configured to process MRI imaging data and the pulse sequence electronics 140 may be configured to generate an MRI imaging sequence, e.g., either a single-shot or a multi-shot MRI scanning pulse sequence. Further, the method 1000 may be executed by the image reconstruction electronics 150 itself. It would also be possible that the method 1000 is at least partially executed by a separate compute unit or an additional computing device, e.g., at a server backend. FIG. 2 illustrates aspects with respect to processing MRI imaging data for a measurement volume including an anatomical region of interest, in particular, masking one or more regions surrounding the anatomical region of interest for each of one or more MRI images obtained from the MRI imaging data. The MRI imaging data are obtained during an MRI exam of a patient for the measurement volume including the anatomical region of interest. For example, the MRI imaging data may be obtained by the MRI scanner 100 of FIG. 1. If the MRI imaging data comprise k-space MRI imaging data, an inverse Fourier transform (IFT), e.g., a fast inverse Fourier transform (IFFT), may be utilized for converting the k-space MRI imaging data to spatial domain MRI imaging data. The one or more regions surrounding the anatomical region of interest are masked based on control data determined for identifying the anatomical region of interest in the MRI imaging data. The anatomical region of interest is ascertained before performing the MRI exam. Details of the method 1000 are described in the following.

Block 1100: ascertaining an anatomical region of interest associated with an MRI exam of a patient. The anatomical region of interest may be ascertained based on at least one of a purpose of the MRI exam, a type of a RF coil used during the MRI exam, and a capability of a physician who will give a diagnosis of the MRI exam.

For example, the anatomical region of interest may be pre-defined or ascertained by a specific clinical/medical practitioner. Alternatively or optionally, the anatomical region of interest may be automatically determined based on one or more indications associated with the MRI exam of the patient. Such indications could be one or more of a purpose of the MRI exam, a type of a RF coil used during the MRI exam, and a capability of a physician who will give a diagnosis of the MRI exam. Details related to automatically ascertaining the anatomical region of interest will be described below.

Based on a purpose of the MRI exam:
As an example, the indication associated with the MRI exam of the patient may comprise a purpose - "orthodontics". Thereby, the anatomical region of interest may be ascertained as to be all teeth, jaw bones, surrounding soft tissues, and ideally all bone structures of the skull for being able to detect therapy relevant landmarks. All other tissues and anatomy and in particular all parts of the brain may be not of interest to a dentist.
As another example, the indication associated with the MRI exam of the patient may comprise another purpose - "temporomandibular joint (TMJ) imaging". Accordingly, the anatomical region of interest may be ascertained as to be the jaw and the TMJ with all bony and joint function related tissues (i.e. cartilage, disk, tendons, muscles) region, and all other tissues and anatomy and in particular all parts of the brain may be regions not of interest.
As a further example, the indication associated with the MRI exam of the patient may comprise a further purpose - "general dental imaging". As such, the anatomical region of interest may be ascertained as to be all teeth, the jaw bone, and all surrounding soft tissue structures up to 1 centimeter (cm) distance, including nerves, vessels, gingiva and etc., and all other regions and in particular all parts of the brain may be regions not of interest.
Optionally or additionally, there may be more specific indications associated with the MRI exam of the patient, e.g., which tooth or which bone should be included in the anatomical region of interest.
It is possible to pre-define or pre-configure a lookup table via which a respective purpose of an MRI exam may be mapped to a corresponding anatomical region of interest. By retrieving keywords of a purpose of an MRI exam, the corresponding anatomical region of interest could be automatically ascertained.

Based on a type of a RF coil:
The anatomical region of interest may be automatically ascertained based on a type of the RF coil which is used to receive MR signals during the MRI exam, e.g., the RF coils 130 shown in FIG. 1. Because each type of RF coil leads to a predetermined selection of an anatomical region of interest, which can be changed manually by the user. The type of RF coil used during an MRI exam can be identified by the coil identifier ( e.g., coil code and/or coil dongle) transmitted to the MR system, e.g., MRI scanner 100 of FIG. 1, depending on the plugged-in RF coil, e.g., the RF coils 130 shown in FIG. 1. The coil identifier may be displayed via the human machine interface 160 of FIG. 1.
As an example, the coil identifier of the RF coil corresponds to an "orthodontics coil" and such a RF coil may indicate that the purpose of the MRI exam is "orthodontics". Thus, the anatomical region of interest may be ascertained as to be all teeth, jaw bones, surrounding soft tissues, and ideally all bone structures of the skull for being able to detect therapy relevant landmarks. All other tissues and anatomy and in particular all parts of the brain may be not of interest to an orthodontist.
As a further example, the coil identifier of the RF coil corresponds to a "dental coil" and such a RF coil may indicate that the purpose of the MRI exam is "general dental imaging". As such, the anatomical region of interest may be ascertained as to be all teeth, the jaw bone, and all surrounding soft tissue structures up to 1 centimeter (cm) distance, including nerves, vessels, gingiva and etc., and all other regions and in particular all parts of the brain may be regions not of interest.
It is also possible to pre-define or pre-configure a lookup table via which a respective type of a RF coil may be mapped to a corresponding anatomical region of interest. By retrieving keywords of a type of a RF coil, the corresponding anatomical region of interest could be automatically ascertained. Further, it would be also possible to pre-define or pre-configure a lookup table indicating correlations among a purpose of an MRI exam, a type of a RF coil, and an anatomical region of interest.

Based on a capability of a physician:
The anatomical region of interest may be automatically ascertained based on a capability of a physician who will give a diagnosis of the MRI exam based on the acquired MRI images. For example, such a capability of a physician may comprise information about the expertise of the physician which may be retrieved or obtained from a database and the anatomical region of interest may be automatically ascertained based on the expertise of the physician.
As an example, the physician who will give a diagnosis of the MRI exam is a normal dentist which may indicate that the purpose of the MRI exam is "general dental imaging". As such, the anatomical region of interest may be ascertained as to be all teeth, the jaw bone, and all surrounding soft tissue structures up to 1 centimeter (cm) distance, including nerves, vessels, gingiva and etc., and all other regions and in particular all parts of the brain may be regions not of interest.
As a further example, the physician who will give a diagnosis of the MRI exam is an orthodontist which may indicate that the purpose of the MRI exam is "orthodontics". Thus, the anatomical region of interest may be ascertained as to be all teeth, jaw bones, surrounding soft tissues, and ideally all bone structures of the skull for being able to detect therapy relevant landmarks. All other tissues and anatomy and in particular all parts of the brain may be not of interest to an orthodontist.

Block 1200: obtaining MRI imaging data for a measurement volume including the anatomical region of interest. After ascertaining the anatomical region of interest, an appropriate scanning pulse sequence may be selected or configured based on the anatomical region of interest, e.g. by means of the human machine interface 160 of FIG. 1. Then, MRI imaging data for a measurement volume including the anatomical region of interest can be obtained or acquired using the scanning pulse sequence. Such scanning pulse sequence can include a series of control commands defining one or more of gradient pulses, RF pulses, readout times, etc.

Block 1300: determining control data for identifying the anatomical region of interest in the MRI imaging data. The control data for identifying the anatomical region of interest may comprise at least one of a contour of the anatomical region of interest, one or more landmarks associated with the anatomical region of interest, a threshold associated with a distance to a respective one of one or more target objects comprised in the anatomical region of interest, and one or more masking images (or mask). The control data may be determined by various approaches which will be explained in detail below.

According to various examples, the control data may be determined based on further MRI imaging data. The method 1000 may further comprise obtaining the further MRI imaging data associated with the anatomical region of interest. For example, the further MRI imaging data may comprise/show more anatomical regions than those of the MRI imaging data obtained at block 1200. Further, the field of view (FOV) of the further MRI imaging data may comprise parts of the anatomical region of interest and parts of the regions surrounding the anatomical region of interest.

In general, the FOV is defined as the size of the two or three-dimensional spatial encoding area of an MRI image and is usually defined in units of mm². The FOV is the square image area that contains the anatomical region of interest to be measured. The smaller the FOV, the higher the resolution and the smaller the voxel size but the lower the measured signal.

According to further examples, the further MRI imaging data may be dedicated to determining the control data.

Optionally or additionally, the further MRI imaging data may comprise localizer data and/or one or more localizer images associated with the anatomical region of interest.

According to various examples, whilst obtaining an MR exam, the radiographer may generate localizer images (also called scout images) to ensure accurate positioning of the patient for the subsequent diagnostic exam. Such localizer images or localizer data may be used for planning MR measurement of an MRI exam, e.g., for setting the FOV of the MR measurement. Additionally or optionally, such localizer images may be used in MR studies to identify the relative anatomical position of a collection of cross-sectional images. A localizer can be acquired as a separate image or it can be dynamically generated. In a localizer image, scout or reference lines may indicate the position of each cross-sectional image. These lines may be "burned into" the image by replacing actual image pixels, stored as a separate overlay, or generated dynamically by, e.g., workstation software. In the last case, clinicians may be able to display specific cross-sectional images by clicking the appropriate scout or reference line in the localizer image. To dynamically generate scout lines, for example, the workstation software may use orientation and slice location information in each cross-sectional image, as well as reference information in the localizer. The localizer data may comprise the scout or reference lines, and/or orientation of the localizer images and/or slice location information of the localizer images.

Generally, localizer data may comprise 3D data with lower resolution and/or with isotropic resolution (e.g., if compared to a subsequent data acquired for images with diagnostic purposes). Therefore, the localizer data may provide a good starting point to identify the anatomical region of interest and/or regions surrounding the anatomical region of interest. However, in some extreme/difficult situations such as TMJ imaging in which brain tissue is located right next to the tissue of interest, namely the TMJ region, this poses a need for a very accurate way to identify the tissue of interest. Consequently, the localizer data may not be enough and additional data with higher resolution may be needed in order to identify the anatomical region of interest and/or the regions surrounding the anatomical region of interest. Such additional data might be tailored to the examination settings for the TMJ imaging. For instance, the additional data might provide additional high-resolution information with regard to the slice orientation in which the TMJ imaging is planned.

According to various examples, the further MRI imaging data may not be stored, e.g., in a database like PACS, with the MRI imaging data obtained at block 1200 because the further MRI imaging data may also include the regions surrounding the anatomical region of interest which are not of interest to a specific clinical/medical practitioner. Accordingly, the method 1000 may further comprise discarding the further MRI imaging data after determining the control data based on the further MRI imaging data.

According to various examples, said determining of the control data based on the further MRI imaging data may comprise determining one or more registration parameters for registering the further MRI imaging data with the MRI imaging data, and determining a contour of the anatomical region of interest based on the further MRI imaging data. The control data may comprise the one or more registration parameters and the contour. For example, the registration parameters may be determined using image registration algorithms available in the prior art such as intensity-based algorithms or featurebased algorithms, transformation models, spatial domain methods, frequency domain methods. Further, the contour of the anatomical region of interest may be also determined using methods available in the prior art such as machineleaning based methods or other segmentation methods. According to this disclosure, the registration parameters may be additionally or optionally used to compensate for the motion of the patient which occurred during the acquisition of the further MRI imaging data and/or during the acquisition of the MRI imaging data (at block 1200).

Optionally or additionally, the further MRI imaging data may be acquired using a different RF coil from that for said obtaining of the MRI imaging data (at block 1200) and/or a different MRI sequence from that for said obtaining of the MRI imaging data (at block 1200). For example, the RF coil (and/or the MRI sequence) for the acquisition of the further MRI imaging data may be set in such a way that regions surrounding the anatomical region of interest may be better visible in the further MRI imaging data than in the MRI imaging data obtained at block 1200. Or alternatively, the RF coil (and/or the MRI sequence) for the acquisition of the further MRI imaging data may be set in such a way that the anatomical region of interest may be better visible in the further MRI imaging data than in the MRI imaging data obtained at block 1200.

According to this disclosure, some other MR imaging techniques may be useful for acquiring the further MRI imaging data which may allow for easily identifying the anatomical region of interest and/or regions surrounding the anatomical region of interest in the further MRI imaging data, i.e., facilitating the identification of the anatomical region of interest and/or regions surrounding the anatomical region. Such MR imaging techniques may comprise at least one of acquisition of T1/T2 signatures, a saturation of brain tissue, echoplanar Imaging (EPI), short measurement time, T2* contrast, and techniques for facilitating skull stripping.

According to various examples, the control data may be determined alternatively or additionally based on the MRI imaging data obtained at block 1200. For example, the control data may be determined based on the further MRI imaging data and the MRI imaging data obtained at block 1200. Alternatively, the control data may be determined based on the MRI imaging data obtained at block 1200 themselves.

Optionally or additionally, the control data may comprise one or more (anatomical) landmarks associated with the anatomical region of interest, which may be determined based on the MRI imaging data. I.e., said determining of the control data may comprise determining, based on the MRI imaging data, the one or more landmarks associated with the anatomical region of interest and the control data may comprise the one or more landmarks. Anatomical landmarks are defined as biologically meaningful loci that can be unambiguously defined and repeatedly located with a high degree of accuracy and precision. The relative location of landmarks provides a spatial map of the relative location of the features that the landmarks represent. Landmark data are useful because there now exist a multitude of methods for the statistical analysis of form using landmark data, but also because more traditional measures like linear distances and angles can be calculated from the landmark data. Traditional anatomical landmarks include foramina for neurovascular bundles, the intersection of sutures and bony processes. For example, such landmarks may comprise one or more specific teeth. Additional landmarks can be defined on the basis of biological knowledge and be used to help identify features that have a direct bearing on research questions.

According to various examples, the anatomical region of interest may comprise one or more target objects, e.g., teeth, and said determining of the control data may comprise determining a threshold associated with a distance to a respective one of the one or more target objects, respectively. The control data may accordingly comprise the threshold. For example, the threshold may be 1 cm to each of the teeth, i.e., the anatomical region of interest may comprise all teeth, the jaw bone, and all surrounding soft tissue structures up to 1 centimeter (cm) distance, including nerves, vessels, gingiva and etc.

Optionally or additionally, the control data may be determined using a trained machine-learning algorithm. For example, the trained machine-learning algorithm may process the MRI imaging data and/or the further MRI imaging data and output the control data characterizing the anatomical region of interest. I.e., said determining of the control data may comprise applying a trained machine-learning algorithm to the MRI imaging data and/or the further MRI imaging data to identify the anatomical region of interest in the MRI imaging data. Such a machine-learning algorithm may be applied during or after the reconstruction of the MRI imaging data or the further MRI imaging data.

According to this disclosure, the trained machine-learning algorithm may be trained based on MRI imaging data acquired by the same MRI scanner for obtaining the MRI imaging data and/or the further MRI imaging data, MRI imaging data acquired by different MRI scanners, synthetic MRI imaging data, or a combination thereof. Further, such machine-learning-based approaches may also allow for usage of MRI imaging data with partial coverage of regions surrounding the anatomical region of interest, e.g., the brain, like it is available from a dedicated dental/TMJ coil not covering the full head.

Optionally or additionally, said determining of the control data may comprise determining one or more masking MRI images. For example, the one or more masking MRI images may be determined based on the control data. For example, the masking MRI image may be determined based on the control data such as at least one of a contour of the anatomical region of interest, one or more landmarks associated with the anatomical region of interest, a threshold associated with a distance to a respective one of one or more target objects comprised in the anatomical region of interest. It is also possible to determine the masking MRI image based on the MRI imaging data and/or the further MRI imaging data using, for example, image segmentation algorithms, and then all pixel values of a region coinciding with the anatomical region of interest are set to 1, and all pixel values of one or more regions respectively coinciding with the one or more regions surrounding the anatomical region of interest are set to 0. I.e., a binary mask can be determined/generated.

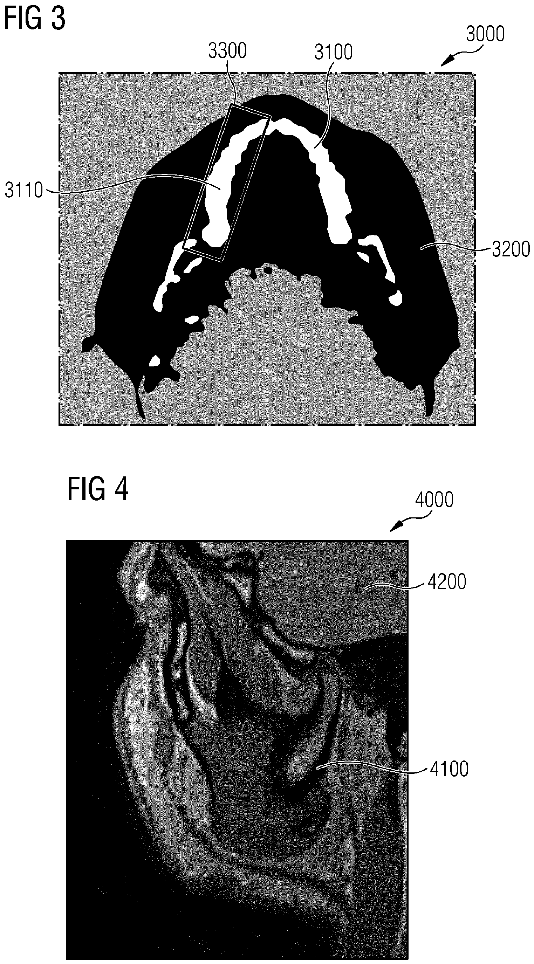

FIG. 3 schematically illustrates an exemplary masking MRI image 3000, i.e., a mask for masking one or more regions surrounding the anatomical region of interest, according to various examples. The masking MRI image 3000 may comprise the anatomical region of interest 3100, i.e., the teeth, and one or more regions 3200 surrounding the anatomical region of interest 3100. In some examples, the masking MRI image 3000 may further comprise a sub-region 3110 of the anatomical region of interest 3100, which may be indicated by, e.g., a bounding box 3300. A clinical/medical practitioner, e.g., a dentist, may be of particular interest to the sub-region 3110.

According to various examples, the masking MRI image 3000 may be displayed in the MRI images using a different, single color to clearly separate the mask pixels from measured pixels. For examples, brain tissue may be displayed as a homogeneous region so that the outline of the brain structure is clearly visible in the displayed image but no diagnostic information may be extracted from the region of the brain. Optionally, the masking MRI image 3000 may include some schematic outline of a brain model to augment the anatomy represented by the unmasked regions, e.g., the anatomical region of interest.

Optionally or additionally, said determining of the control data may be further based on imaging data associated with the anatomical region of interest which are acquired using one or more other imaging modalities, e.g., X-ray, computed tomography, ultrasound and etc. For example, given a 3D MRI image of a sub-volume of the jaw, and cone beam computed tomography images of the mandibular region which have been already segmented, inline registration of the datasets is performed to identify the mandible region, including the teeth, and subsequently define the anatomical region of interest during image reconstruction.

Optionally or additionally, said determining of the control data may be further based on a difference between signal-to-noise ratio (SNR) of the anatomical region of interest and SNR of one or more regions surrounding the anatomical region of interest.

It is conceivable that the anatomical region of interest and the one or more regions surrounding the anatomical region of interest may have different SNR. I.e., the anatomical region of interest may have a higher/lower SNR than that of the one or more regions surrounding the anatomical region of interest because the RF coil may be closer/farther to the anatomical region of interest than to the one or more regions surrounding the anatomical region of interest. Further, the patient may be positioned in such a way that the anatomical region of interest may be closer to the imaging region of the MRI scanner than the one or more regions surrounding the anatomical region of interest, e.g., in case a dedicated MRI scanner or a dedicated RF coil is used for imaging the anatomical region of interest.

Optionally or additionally, said determining of the control data may be further based on one or more optical images including the anatomical region of interest. The method 1000 may further comprise capturing the one or more optical images including the anatomical region of interest, and registering each of the one or more optical images with the MRI imaging data.

For example, the optical images may be obtained/captured by one or more cameras mounted on the MRI scanner 100 of FIG. 1. Thus, it is possible to combine camera-based localization, for example, where the measurement volume including the anatomical region of interest may be defined without the acquisition of the further MRI imaging data, and the measurement volume including the anatomical region of interest may cover a part of, e.g., the subject head/jaw area, the acquired images can be matched with a digital (synthetic) twin of the exam protocol that simulates the entire volume. Thus, in matching the measurement volumes (acquired and synthetic), where the synthetic data may contain classified anatomical areas, the anatomical region of interest can be identified and processed appropriately.

Block 1400: based on the control data, masking one or more regions surrounding the anatomical region of interest for each of one or more MRI images obtained from the MRI imaging data.

After determining the control data for identifying the anatomical region of interest, for each of one or more MRI images obtained from the MRI imaging data, one or more regions surrounding the anatomical region of interest are masked out. Thus, the one or more obtained/reconstructed MRI images can be adapted in a suitable way so that the one or more regions surrounding the anatomical region of interest can be masked out, e.g., by setting pixel/voxel values of the one or more regions surrounding the anatomical region of interest to be 0 or by cropping the one or more regions surrounding the anatomical region of interest. For instance, such cropping may be implemented by reducing the matrix size of the one or more obtained/reconstructed MRI images such that the pixels/voxels of the one or more regions surrounding the anatomical region of interest can be removed.

According to various examples, when the control data (determined at block 1300) may comprise the one or more registration parameters and the contour, said masking of the one or more regions surrounding the anatomical region of interest may comprise registering the contour with each of the one or more MRI images based on the one or more registration parameters and blurring, removing, or fading out the one or more regions outside the contour for each of the one or more MRI images.

Optionally or additionally, the method 1000 may further comprise selecting a reference image from the one or more MRI images and adjusting, in the reference image, a position of and/or a size of the contour of the anatomical region of interest. Said registering of the contour with each of the one or more MRI images may comprise registering the adjusted contour with each of the one or more MRI images based on the one or more registration parameters.

For example, after obtaining several slices of MRI imaging data for the measurement volume including the anatomical region of interest, several slices of MRI images may be obtained/reconstructed. Then, a reference image can be selected from the several slices of MRI images and the position of and/or the size of the contour of the anatomical region of interest can be adjusted based on the position of and/or the size of the anatomical region of interest in the selected reference image. Accordingly, Said registering of the contour with each of the one or more MRI images may be based on the adjusted contour. Therefore, the contour determined based on the further MRI imaging data can be tailored/adjusted based on the MRI imaging data and thereby the one or more regions surrounding the anatomical region of interest can be masked out more precisely.

According to various examples, when the control data (determined at block 1300) may comprise one or more landmarks associated with the anatomical region of interest, said masking of the one or more regions surrounding the anatomical region of interest may be based on the one or more landmarks and/or a pre-defined model of the anatomical region of interest.

According to further examples, when the control data (determined at block 1300) may comprise one or more masking MRI images, said masking of the one or more regions surrounding the anatomical region of interest may be based on the one or more masking MRI images, e.g., masking MRI image 3000 of FIG. 3.

According to still further examples, the control data may comprise a combination of the one or more registration parameters and the contour, the one or more landmarks associated with the anatomical region of interest, and one or more masking MRI images said masking of the one or more regions surrounding the anatomical region of interest may be based on the combination. Thus, the one or more regions surrounding the anatomical region of interest can be masked more precisely. The more the control data, the more precisely the masking of the one or more regions surrounding the anatomical region of interest.

Additionally or optionally, after masking one or more regions surrounding the anatomical region of interest for each of one or more MRI images obtained from the MRI imaging data, the method 1000 may further comprise obtaining a graphical representation associated with the anatomical region of interest based on the one or more masked MRI images.

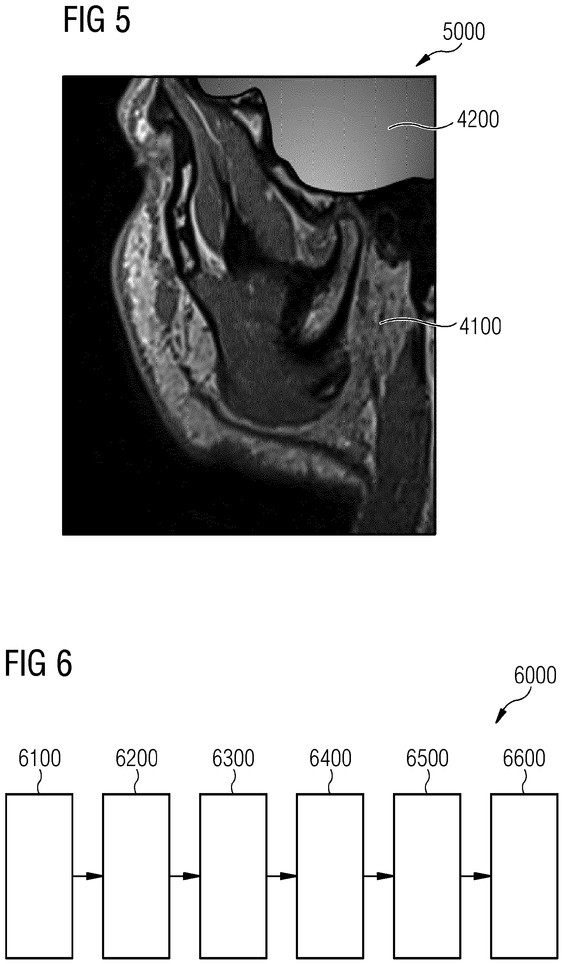

FIG. 4 schematically illustrates an exemplary MRI image 4000 obtained from the MRI imaging data. The MRI image 4000 may comprise an anatomical region of interest 4100 and one region 4200 surrounding the anatomical region of interest 4100. The region 4200 surrounding the anatomical region of interest 4100 comprises a part of the brain of a patient and the anatomical region of interest 4100 comprises parts of the head of the patient other than the brain. FIG. 5 schematically illustrates an exemplary masked MRI image 5000 according to various examples. The masked MRI image 5000 is obtained by masking the region 4200 surrounding the anatomical region of interest 4100 in the MRI image 4000 of FIG. 4.

According to various, multiple 2D MRI images, i.e., multiple slices, may be obtained/reconstructed from the MRI imaging data and for each of the multiple 2D MRI images, the region 4200 surrounding the anatomical region of interest 4100 can be masked out using techniques disclosed herein. Then, based on respective masked MRI images, a graphical representation associated with the anatomical region of interest may be obtained/generated, e.g., using medical visualization techniques available in the prior art.

Optionally, the method 1000 may further comprise storing the control data together with the graphical representation and/or the one or more masked MRI images. For example, the control data indicating the masked region (i.e., the one or more regions surrounding the anatomical region of interest), instead of being encoded directly into the MRI image, could be stored within the MRI image header, e.g. DICOM presentation state, and depending on the image reading environment and reader, be overlaid or not on the MRI image.

Optionally, the method 1000 may further comprise adjusting imaging parameters associated with the MRI exam based on the graphical representation. For example, the imaging parameters may comprise parameters associated with the measurement volume.

For example, if the graphical representation may comprise parts of but not all of the anatomical region of interest in a plane, e.g., the x-y plane of FIG. 1, for example, the FOV may be adjusted by modifying the MRI sequence.

The techniques disclosed herein, e.g., the method 1000, can automatically, rapidly, and accurately isolate or segregate an anatomical region of interest for a specific clinical/medical practitioner from surrounding anatomical regions by masking the one or more regions surrounding the anatomical region of interest for each of the one or more MRI images obtained from MRI imaging data. Thus, the workload for the specific clinical/medical practitioner to diagnose the acquired images can be reduced and risks for the specific clinical/medical practitioner to miss pathologies in regions which she/he is not trained to diagnose can be reduced.

In general, the masking of the one or more regions surrounding the anatomical region of interest can be made selectable in the user interface. It may be a decision made at the beginning of each individual exam and last throughout the exam. It may also be an option to deactivate the masking in the course of the exam or for individual scans only. It may also be possible to activate the masking as a permanent default setting for a specific MRI scanner, e.g., a dental MRI scanner, in such a way that the normal operator is not allowed to deactivate it. Besides the selection of if the masking shall be applied or not, it may also be configurable in its behavior by either the user or an admin.

Optionally, the properties of the masking may also depend on sub-indications. For example, in an orthodontics scan with a larger FOV, the masking may not cover some landmarks which are specifically required for an orthodontics assessment. For any other indication, related to the teeth only, the masking may be more restrictive.

According to various examples, the operator of the MRI scanner may be enabled to draw on a reference image to indicate which parts of the presented mask shall be cropped; shift/zoom the mask on a reference image to move it away from a region of interest; control a parameter which resembles the balance between a generous coverage of unwanted structures (i.e., the one or more regions surrounding the anatomical region of interest), risking the coverage of relevant anatomy at the borders (i.e., the anatomical region of interest), and on the other hand a restrictive coverage of unwanted structures risking the disclosure of unwanted pixels as part of the anatomical region of interest. This parameter may be controlled stepwise or by a continuous slider or numerical value.

The parameter may adapt to the exact target anatomy, e.g., position within the brain, or based on the distance to identified landmarks.

The parameter may also be varied according to the local properties of the reference image, e.g. if the SNR in a region is low (e.g. deep brain structures with surface coil acquisition), the safety margin may be increased, whereas in regions of high SNR the safety margin may be lowered to draw an exact line between brain and dental target anatomy (e.g. in TMJ region).

According to various examples, the masking (e.g., masking MRI images) may be determined based on the first localizer image, which may not be shown to the operator, or it may be shown with the mask already applied to it.

According to this disclosure, the masking may be generated based on the segmentation of the initial images within an exam, i.e., based on the MRI imaging data obtained at block 1200. Another option is to generate the mask based on the detection of landmarks and a brain model which may be adapted to the individual anatomy based on the identified landmarks.

FIG. 6 schematically illustrates an exemplary workflow 6000 by utilizing the method 1000 of FIG. 2 according to various examples. The workflow 6000 pertains to performing an MRI scan, in which one or more regions surrounding an anatomical region of interest for MRI images are masked out based on control data determined based on further MRI imaging data, e.g., localizer images obtained in a scout scan. Details with respect to the workflow 6000 will be described below.

Step 6100: ascertaining an anatomical region of interest associated with an MRI exam of a patient. Step 6100 is the same as block 1100 of FIG. 1.

Step 6200: obtaining one or more localizer images associated with the anatomical region of interest as explained above.

Step 6300: determining control data for identifying the anatomical region of interest based on the one or more localizer images. Step 6300 may be the same as part of block 1300 of FIG. 1.

Step 6400: obtaining MRI imaging data for a measurement volume including the anatomical region of interest. Step 6400 is the same as block 1200 of FIG. 1.

Step 6500: based on the control data, masking one or more regions surrounding the anatomical region of interest for each of one or more MRI images obtained from the MRI imaging data. Step is the same as block 1400 of FIG. 1. Step 6500 may be performed simultaneously with step 6400.

Step 6600: performing post-processing. For example, the post-processing may comprise at least one of obtaining a graphical representation associated with the anatomical region of interest based on the one or more masked MRI images, storing the control data together with the graphical representation, and adjusting imaging parameters associated with the MRI exam based on the graphical representation.

It is conceivable that in the case that further MRI imaging data, e.g. localizers, are obtained at the start of an MRI exam, e.g., a dental examination, these images may be displayed to the operator to assist in planning further image acquisitions, i.e., the MRI imaging data, or displayed as part of a quality control step to verify automatically prescribed image volumes, e.g., the measurement volume including the anatomical region of interest. However, it may not be possible to segment these images adequately due to insufficient image contrast. Nevertheless, for example, given that a dental examination may typically require the acquisition of volumes of the jaw, where these volumes are oriented relative to the teeth, the localizer images can be converted to a binary masked image, whereby the teeth are obviously different to the rest of the anatomy. Thus, this method uses the fact that teeth are, in most standard sequences, MR-invisible.

According to some examples, it would also be possible that the localizer images are output in a user interface to a user; the masking can be applied directly in the localizer images. I.e., at a relatively early stage of the image acquisition workflow, it is possible to apply the masking.

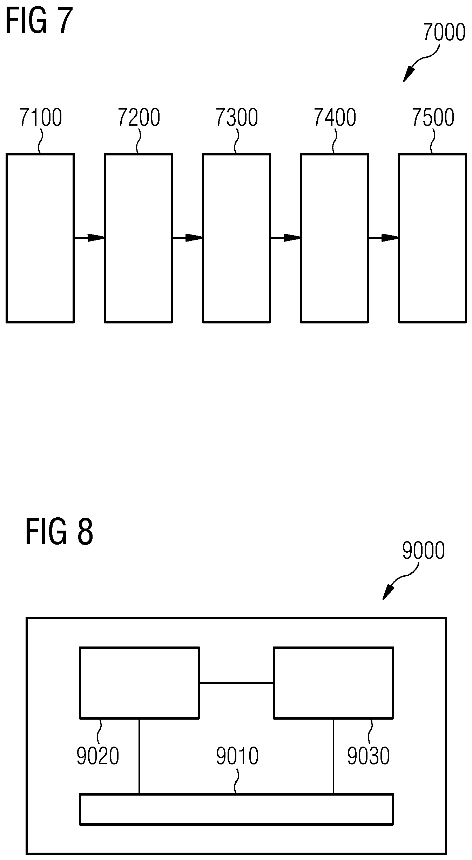

FIG. 7 schematically illustrates a further exemplary workflow 7000 by utilizing the method 1000 of FIG. 2 according to various examples. The workflow 7000 pertains to performing an MRI scan, in which one or more regions surrounding an anatomical region of interest for MRI images are masked out based on control data determined based on MRI imaging data for a measurement volume including the anatomical region of interest, i.e., without a scout scan. Details with respect to the workflow 7000 will be described below.

Step 7100: ascertaining an anatomical region of interest associated with an MRI exam of a patient. Step 7100 is the same as block 1100 of FIG. 1.

Step 7200: obtaining MRI imaging data for a measurement volume including the anatomical region of interest. Step 7200 is the same as block 1200 of FIG. 1.

Step 7300: based on the obtained MRI imaging data, determining control data for identifying the anatomical region of interest in the MRI imaging data.

Step 7400: based on the control data, masking one or more regions surrounding the anatomical region of interest for each of one or more MRI images obtained from the MRI imaging data. Step 7400 is the same as block 1400 of FIG. 1.

Step 7500: performing post-processing. Step 7500 is the same as step 6600 of FIG. 6.

FIG. 8 is a block diagram of a computing device 9000 according to various examples. The computing device 9000 may comprise a processor 9020, a memory 9030, and an input/output interface 9010. The processor 9020 is configured to load program code from the memory 9030 and execute the program code. Upon executing the program code, the processor 9020 performs the method 1000 for processing MRI imaging data for a measurement volume including an anatomical region of interest. Further, the computing device 9000 may facilitate the workflows 6000 and 7000.

Referring to FIG. 1 again, the MRI scanner 100 may further comprise the computing device 9000 configured to perform the method 1000. The computing device 9000 may be the image reconstruction electronics 150 and/or the pulse sequence electronics 140. I.e., the computing device 9000 may be embedded in or connected with the MRI scanner 100, and thereby the MRI scanner 100 may be also configured to perform the method 1000.

Summarizing, techniques have been described that facilitate masking one or more regions surrounding an anatomical region of interest for MRI images. The techniques disclosed herein adapt/process the images acquired using an MR scanner, e.g., a dental MR scanner so that the images may exclusively show the region of the patient, which is of interest to a dentist or a dental practitioner. I.e., the techniques disclosed herein can automatically, rapidly, and accurately isolate or segregate an anatomical region of interest for a specific clinical/medical practitioner from surrounding anatomical regions. Thus, the workload for the specific clinical/medical practitioner to diagnose the acquired images can be reduced and risks for the specific clinical/medical practitioner to miss pathologies in regions which she/he is not trained to diagnose can be reduced. For example, a dentist may not be trained for diagnosing pathologies in the brain.

Although the disclosure has been shown and described with respect to certain preferred embodiments, equivalents and modifications will occur to others skilled in the art upon the reading and understanding of the specification. The present disclosure includes all such equivalents and modifications and is limited only by the scope of the appended claims.

Further, the techniques disclosed herein can be also applied to 3-D MRI imaging data.

As an alternative, instead of (or in addition to) identifying the anatomical region of interest and/or regions surrounding the anatomical region of interest in the acquired MRI imaging data or reconstructed MRI images, the acquisition of the MRI imaging data may be tailored in such a way that the acquisition of data from the regions surrounding the anatomical region of interest is suppressed so that the acquired MRI imaging data only (or mostly) show data from the anatomical region of interest. For example, this may be achieved using so-called "spatial saturation bands". It is possible to effectively suppress the signal in the area defined by the geometry of the saturation band. Briefly, the method involves the application of selective RF pulses that are designed to suppress magnetization.

## Claims

1. A computer-implemented method (1000), comprising:
- ascertaining (1100) an anatomical region of interest (3100) associated with a magnetic resonance imaging, MRI, exam of a patient;
- obtaining (1200) MRI imaging data for a measurement volume including the anatomical region of interest (3100);
- determining (1300) control data for identifying the anatomical region of interest (3100) in the MRI imaging data;
- based on the control data, masking (1400) one or more regions (3200) surrounding the anatomical region of interest (3100) for each of one or more MRI images (4000) obtained from the MRI imaging data.

2. The computer-implemented method of claim 1, wherein the anatomical region of interest (3100) is ascertained based on at least one of a purpose of the MRI exam, a type of a radiofrequency, RF, coil used during the MRI exam, and a capability of a physician who will give a diagnosis of the MRI exam.

3. The computer-implemented method of claim 1 or 2, further comprising:
- obtaining further MRI imaging data associated with the anatomical region of interest (3100);
wherein said determining of the control data is based on the further MRI imaging data.

4. The computer-implemented method of claim 3, wherein the further MRI imaging data comprise localizer data and/or one or more localizer images associated with the anatomical region of interest (3100).

5. The computer-implemented method of claim 3,
wherein said determining of the control data based on the further MRI imaging data comprises:
- determining one or more registration parameters for registering the further MRI imaging data with the MRI imaging data;
- determining a contour of the anatomical region of interest (3100) based on the further MRI imaging data;
wherein the control data comprises the one or more registration parameters and the contour;
wherein said masking of the one or more regions (3200) surrounding the anatomical region of interest (3100) comprises:
- registering the contour with each of the one or more MRI images based on the one or more registration parameters;
- blurring, removing, or fading out the one or more regions outside the contour for each of the one or more MRI images.

6. The computer-implemented method of claim 5, further comprising:
- selecting a reference image from the one or more MRI images;
- adjusting, in the reference image, a position of and/or a size of the contour of the anatomical region of interest (3100);
wherein said registering of the contour with each of the one or more MRI images comprises:
- registering the adjusted contour with each of the one or more MRI images based on the one or more registration parameters.

7. The computer-implemented method of any one of claims 3 to 6, wherein the further MRI imaging data are acquired using a different RF coil from that for said obtaining of the MRI imaging data and/or a different MRI sequence from that for said obtaining of the MRI imaging data.

8. The computer-implemented method of any one of the preceding claims, wherein said determining of the control data comprises:
- determining, based on the MRI imaging data, one or more landmarks associated with the anatomical region of interest (3100);
wherein the control data comprises the one or more landmarks;
wherein said masking of the one or more regions (3200) surrounding the anatomical region of interest (3100) is based on the one or more landmarks and/or a pre-defined model of the anatomical region of interest (3100).

9. The computer-implemented method of any one of the preceding claims,
wherein the anatomical region of interest (3100) comprises one or more target objects and said determining of the control data comprises:
- determining a threshold associated with a distance to a respective one of the one or more target objects, respectively;
wherein the control data comprises the threshold.

10. The computer-implemented method of any one of the preceding claims, wherein said determining of the control data comprises:
- applying a trained machine-learning algorithm to the MRI imaging data to identify the anatomical region of interest (3100) in the MRI imaging data.

11. The computer-implemented method of any one of the preceding claims, wherein said determining of the control data comprises:
- determining one or more masking MRI images (3000);
wherein said masking of the one or more regions (3200) surrounding the anatomical region of interest (3100) is based on the one or more masking MRI images (3000).

12. The computer-implemented method of any one of the preceding claims, wherein said determining of the control data is further based on imaging data associated with the anatomical region of interest (3100) which are acquired using one or more other imaging modalities.

13. The computer-implemented method of any one of the preceding claims, wherein said determining of the control data is further based on a difference between signal-to-noise ratio, SNR, of the anatomical region of interest (3100) and SNR of the one or more regions (3200) surrounding the anatomical region of interest (3100).

14. The computer-implemented method of any one of the preceding claims, further comprising:
- capturing one or more optical images including the anatomical region of interest (3100); and
- registering each of the one or more optical images with the MRI imaging data;
wherein said determining of the control data is further based on the one or more optical images.

15. The computer-implemented method of any one of the preceding claims, further comprising:
- obtaining a graphical representation associated with the anatomical region of interest (3100) based on the one or more masked MRI images.

16. The computer-implemented method of claim 15, further comprising:
- storing the control data together with the graphical representation.

17. The computer-implemented method of claim 15 or 16, further comprising:
- adjusting imaging parameters associated with the MRI exam based on the graphical representation.

18. The computer-implemented method of claim 17, wherein the imaging parameters comprise parameters associated with the measurement volume.

19. A computing device (9000), the device (9000) comprising a processor (9020) and a memory (9030), wherein upon loading and executing program code from the memory (9030), the processor (9020) is configured to perform the method (1000) of any one of the preceding claims.

20. An MRI scanner (100), the MRI scanner (100) comprising the computing device (9000) of claim 19.
